# EUROPEAN PATENT SPECIFICATION

(11) **EP 1 621 216 B1**
(45) Date of publication and mention of the grant of the patent: **28.11.2007**
(21) Application number: 05015990.4
(22) Date of filing: 22.07.2005
(51) Int. Cl.: A61L 27/18, A61L 27/56, A61L 27/58, C08L 67/04

(54) **Bioabsorbable polymeric granular porous bone filling material and production method thereof**
Bioabsorbierbares granulöses poröses Polymerknochenfüllmaterial und dessen Herstellung
Matériau polymérique bioabsorbable sous forme granuleuse et poreuse pour charge osseuse et sa préparation

(30) Priority: 26.07.2004 JP 2004216887
(43) Date of publication of application: 01.02.2006
(73) Proprietor: GC Corporation, Tokyo 174-8585 (JP)
(72) Inventor: Yamamoto, Katushi, Tokyo 174-8585 (JP); Yamanaka, Katsuyuki, Tokyo 174-8585 (JP); Suda, Youko, Tokyo 174-8585 (JP); Kaneko, Tadashi, Tokyo 174-8585 (JP)
(74) Representative: Müller-Boré & Partner Patentanwälte

(56) References cited:
- EP-A- 1 234 587
- EP-A- 1 541 182
- WO-A-00/62829
- US-A- 5 866 155

## Description

The present invention relates to a bioabsorbable granular porous bone filling material and a production method thereof. This material is used for filling a defect part after removing a lesion due to osteoma, osteomyelitis or the like and after grafting a self bone, and also used for reinforcing, filling or the like of a jawbone at the time of embedding a dental implant.

As an artificial bone material, an inorganic material, such as hydroxyapatite ceramics, tricalcium phosphate ceramics, a calcium phosphate-based glass or the like, has been conventionally used. As a shape of the artificial bone material, a rod or block shape is general, but a granular shape is also widely used for filling the defect part, as this granular shape can be filled to details of the defect part, and applied to an arbitrary-shaped defect part.

As a bone filling material for filling the defect part, a granular material obtained by pulverizing the above-mentioned inorganic material (for example, refer to Japanese Patent Laid Open No. 1994-339519, 2002-58735, 2004-24319) has been used. However, although the inorganic material, such as the hydroxyapatite ceramics or the like, is a similar component of the bone, it has a problem that a foreign substance remains in a body since it is a nonabsorbable material with respect to a living body.

A bone filling material originating in an animal, such as a demineralized freeze dried cow bone allograft or the like, is used in addition to the inorganic material. However, although the bone filling material originating in the animal has excellent bio-compatibility, it has a problem in safety with respect to an unknown pathogen when it is used as a medical supply with respect to a human body. Such an unknown pathogen is represented by BSE (bovine spongiform encephalopathy).

For solving these problems, it can be considered to use a bioabsorbable (hereinafter, it is also said to as a biodegradable) polymer material which exists for bone filling until the bone tissues is reproduced, and is absorbed by the living body to disappear when the reproducing of the bone tissues is finished. However, even when a biodegradable polymer block is only pulverized to make the granular bone filling material, the absorption to the living body takes time beyond the need in many cases, and this problem cannot be solved by only changing the diameter of the granule.

Then, a material with a porous structure called as a foam type, a sponge type or the like provided with many holes having a diameter of about 180 to 500 µm in the biodegradable polymer material to increase the absorption efficiency to the living body, is also used (for example, refer to Japanese Patent Laid Open No. 2002-20523, 2002-146084). However, the conventional production method of the biodegradable polymer material having the porous structure is to produce the material in a mold having a certain size, so that there are problems that only a block in the foam type, the sponge type or the like, is produced, and thus the granular material cannot be obtained. That is, since the block shaped material is a soft material having the porous structure, there is a technical limit for making it fine at the medical treatment place, and the infection possibility is also increased. Further, it is very hard to industrially pulverize the biodegradable polymer material which is the soft material having the porous structure, and the yield in pulverizing is remarkably low.

Furthermore, since these biodegradable polymer materials are used for the tissue engineering by mostly seeding a living cell, there is a problem that these materials are too soft for filling material and inferior in shaping property, for the reason that these materials have the porous structure in the foam type or the sponge type with the holes having about 180 to 500 µm diameter, which are larger than the sizes of the cell.

The objective of the present invention is to provide a bioabsorbable granular porous bone filling material and the production method thereof to solve the problems in the above-mentioned conventional techniques. The bioabsorbable granular porous bone filling material comprises the bioabsorbable polymer material, which has a small hole structure having a hole diameter of 5 to 50 µm, and has a particle diameter of 100 to 3000 µm for easily carrying out the filling operation to the defect part of a patient.

The earnest work was carried out in order to solve the above-mentioned problems and, as a result of this, the followings were found out to solve the above-mentioned problems. The bioabsorbable polymer material having the hole diameter of 5 to 50 µm can be obtained effectively by the process comprising, freezing a solution, where the bioabsorbable polymer is dissolved with an organic solvent and drying it to remove the organic solvent. Further, the bioabsorbable polymer material, which is the soft material having a porous structure, can be industrially pulverized to have the particle diameter of 100 to 3000 µm effectively by the processes of approximate-uniformly mixing a particle-shaped material having a desired diameter, can be dissolved in the liquid which does not dissolve the bioabsorbable polymer with the solution, where the bioabsorbable polymer is dissolved with an organic solvent, and making thus produced polymer material into the hard material as compared with the bioabsorbable polymer material having the conventional porous structure, by freezing and drying and after pulverizing to produce the bioabsorbable granular porous bone filling material having the particle diameter of 100 to 3000 µm. Then, the present invention is completed.

That is, the present invention relates to the bioabsorbable granular porous bone filling material comprising the bioabsorbable polymer material which has the small hole structure having the hole diameter of 5 to 50 µm, and has a particle diameter of 100 to 3000 µm and the production method of the bioabsorbable granular porous bone filling material having the particle diameter of 100 to 3000 µm , comprising, approximate-uniformly mixing a particle-shaped material having a particle diameter of 100 to 2000 µm with the solution, where the bioabsorbable polymer is dissolved with an organic solvent, freezing it, drying it, removing thus the organic solvent, thereby, to produce the polymer material which has the small hole structure having the hole diameter of 5 to 50 µm and contains the particle-shaped material, pulverizing it, dissolving it in a liquid to remove the particle-shaped material, and passing it through the particle diameter sorting apparatus such as sieve, where the particle-shaped material cannot be dissolved with the organic solvent but can be dissolved with the liquid which does not dissolve the bioabsorbable polymer.

In the bioabsorbable granular porous bone filling material and the production method thereof, as for the bioabsorbable polymer for the polymer material, it is preferable to select at least one kind from polyglycolic acid, polylactic acid, a copolymer of lactic acid and glycolic acid, poly- ε- caprolactone, a copolymer of lactic acid and ε- caprolactone, polyamino acid, polyortho ester, and a copolymer of those. Further, it is preferable that the weight average molecular weight of the bioabsorbable polymer is 5000 to 2000000.

Further, in the production method of the bioabsorbable granular porous bone filling material, it has been found out that at least one kind selected from chloroform, dichloromethane, carbon tetrachloride, acetone, dioxane, and tetrahydrofuran is preferably used for the organic solvents, that a water-soluble organic and/or inorganic salt is preferably used for the particle-shaped material, that water is preferably used for the liquid which dissolves the particle-shaped material and does not dissolve the bioabsorbable polymer, and that the concentration of the bioabsorbable polymer to the organic solvent is preferably 1 to 20 wt.%, and the concentration of the particle-shaped material to the organic solvent is preferably 1.0 to 1.5 g/cm³ at the time of producing the polymer material.

The bioabsorbable granular porous bone filling material according to the present invention comprises the soft material having porous structure, which is hardly pulverized in general, but is a new bioabsorbable granular porous bone filling material, which has a particle diameter for easily carrying out the filling operation to the defect part of a patient.

Further, the production method of bioabsorbable granular porous bone filling material according to the present invention is the method for efficiently producing the bioabsorbable granular porous bone filling material, in which the conventional polymer material having the porous structure is impossible to be pulverized because of being the soft material. Further, the produced bioabsorbable granular porous bone filling material has the high bioabsorbency, since it has the small hole structure as compared with the conventional bone filling material, which is produced by pulverizing the block of the bioabsorbable polymer material not having the hole to make the granular material.

Figure 1 is a scanning electron microscope photograph of a bioabsorbable granular porous bone filling material produced in Example 1.

Figure 2 is a scanning electron microscope photograph of a bioabsorbable granular porous bone filling material produced in Example 4.

In the present invention, the bioabsorbable polymer for the bioabsorbable granular porous bone filling material is safe for the living body, and can keep the shape in the body during a fixed period. Further, when the solution dissolving the particle-shaped material, which is mentioned below, does not have solubility for the polymer, the polymer can be used without especial limitation. For example, at least one kind selected from polyglycolic acid, polylactic acid, a copolymer of lactic acid and glycolic acid, poly- ε- caprolactone, a copolymer of lactic acid and ε- caprolactone, polyamino acid, polyortho ester, and a copolymer of those can be used. In these materials, polyglycolic acid, polylactic acid, and the copolymer of lactic acid and glycolic acid are the most preferable since these are recognized from U.S. Food and Drug Administration (FDA) as the polymer being harmless to a human body, and have actual results. It is preferable that the weight average molecular weight of the bioabsorbable polymer is 5000 to 2000000, and more preferably 10000 to 500000.

In the method of the present invention, the bioabsorbable polymer is dissolved with the organic solvent in the producing process. While the organic solvent is used suitably selecting with the polymer material to be used, at least the one kind selected from chloroform, dichloromethane, carbon tetrachloride, acetone, dioxane, and tetrahydrofuran is preferably used in general. In the dissolving process, a heat treatment or an ultrasonic treatment may be used together. While the concentration of the bioabsorbable polymer is not limited especially if this polymer can be dissolved uniformly in the organic solvent, preferable concentration is 1 to 20 wt.% in the organic solvent.

In the method of the present invention, the particle-shaped material having the particle diameter of 100 to 2000 µm is approximate-uniformly mixed with the organic solvent in which the bioabsorbable polymer is dissolved, in the producing process. This particle-shaped material is not dissolved with the organic solvent in which the bioabsorbable polymer is dissolved and is dissolved with the liquid which does not dissolve the bioabsorbable polymer. The particle-shaped material can exist in the solid state in the polymer material, until the polymer material is pulverized to become the granular material. The polymer material is produced by approximate-uniformly mixing the particle-shaped material with the solution in which the bioabsorbable polymer is dissolved, freezing it, drying it, and removing the organic solvent. Thus, the polymer material contains the particle-shaped material and has the small hole structure having the hole diameter of 5 to 50 µm. Further, the particle-shaped material can be rapidly dissolved and removed with the liquid, which does not dissolve the bioabsorbable polymer, after pulverizing. The polymer material becomes hard since the particle-shaped material exists in the solid state (the particle state) in the polymer material, so that the pulverization becomes easy, and thus, the granule having the arbitrary particle diameter can be easily produced.

As the method for approximate-uniformly mixing the particle-shaped material with the organic solvent in which the bioabsorbable polymer is dissolved, there is a method of adding the particle-shaped material into the organic solvent in which the bioabsorbable polymer is dissolved, stirring and mixing it if necessary, and taking it into a mold, a method of pouring the organic solvent in which the bioabsorbable polymer is dissolved into a mold where the particle-shaped material is already put in, or a method of pouring the particle-shaped material into a mold where the organic solvent in which the bioabsorbable polymer is dissolved is already put in.

It is necessary that the particle diameter of the particle-shaped material is 100 to 2000 µm, and an aggregated granular crystalline particle may be used if it is a crystalline material. If the particle diameter of the particle-shaped material is less than 100 µm , the size of the polymer among the particle-shaped materials in the produced polymer material becomes small, so that the yield in pulverizing the polymer material having the objective particle diameter of 100 µm or more may be decreased. If the particle diameter is more than 2000 µm, the amount of the particle-shaped materials in the produced polymer material is increased, so that the yield in pulverizing may be decreased. It is more preferable that the particle diameter of the particle-shaped material is 200 to 1000 µm.

Further, it is preferable that the blending amount of the particle-shaped material to the organic solvent in which the bioabsorbable polymer is dissolved is 1.0 to 1.5 g/cm³. If the blending amount is less than 1.0 g/cm³, the effect for hardening the produced polymer material is hardly obtained. If this amount is more than 1.5 g/cm³, the ratio of the polymer in the produced polymer material is decreased, so that the production yield may be decreased. More preferably, the blending amount is 1.0 to 1.25 g/cm³.

As for the particle-shaped material used in the present invention, it is not limited especially if this material has the characteristic that the material is not dissolved with the organic solvent in which the bioabsorbable polymer is dissolved, and dissolved with the liquid which does not dissolve the bioabsorbable polymer. However, preferable particle-shaped material are water soluble organic and/or inorganic salts like an inorganic salt such as sodium chloride, potassium chloride, calcium chloride or the like, an ammonium salt such as ammonium chloride or the like, or an organic salt such as citric acid trisodium or the like, because these materials can use cheap water as the liquid which does not dissolve the bioabsorbable polymer. Sodium chloride, potassium chloride and citric acid trisodium are especially preferable as these particle-shaped materials are easily available and have little damage to a human body.

In the method of the present invention, the polymer material containing the particle-shaped material and the small hole structure having the hole diameter of 5 to 50 µm is produced by approximate-uniformly mixing the particle-shaped material with the solution of the bioabsorbable polymer being dissolved with the organic solvent, freezing it using a freezer or liquid nitrogen, drying it, and removing the organic solvent. However, the operation for removing the organic solvent changes with the organic solvent. For example, if the solvent is an organic solvent having high volatility, the solvent is only kept at the room temperature, but the solvent is dried under a reduced pressure using a vacuum dryer in general.

In the method of the present invention, the bioabsorbable granular porous bone filling material having the particle diameter of 100 to 3000 µm is obtained by pulverizing the polymer material, removing the particle-shaped material by dissolving it with the liquid which does not dissolve the bioabsorbable polymer, and passing it through the sieve. At this time, although a method for removing the particle-shaped material changes with the material, if the water soluble organic and/or inorganic salt such as the sodium chloride, the potassium chloride, the calcium chloride, the ammonium chloride, the citric acid trisodium or the like is used as mentioned above, the material can be removed easily and safely with water.

In this case, the reason why the particle diameter of the bioabsorbable granular porous- bone filling material is 100 to 3000 µm is the following. If the particle diameter is less than 100 µm, the particle diameter is too small, so that the bone forming is prevented by a slight movement phenomenon caused by the filling material being not stable and not fixed at the filling part. Further, a phagocytic cell such as a foreign body giant cell or the like eats the bioabsorbable granular porous bone filling material having the small diameter to cause the inflammatory reaction. Thus, it is not preferable. If the particle diameter is more than 3000 µm, a space among the bioabsorbable granular porous bone filling materials becomes large, so that an epithelial cell or the like invades into the space in the bioabsorbable granular porous bone filling material before the bone reproduction. Thus, it is not preferable.

### <Example 1>

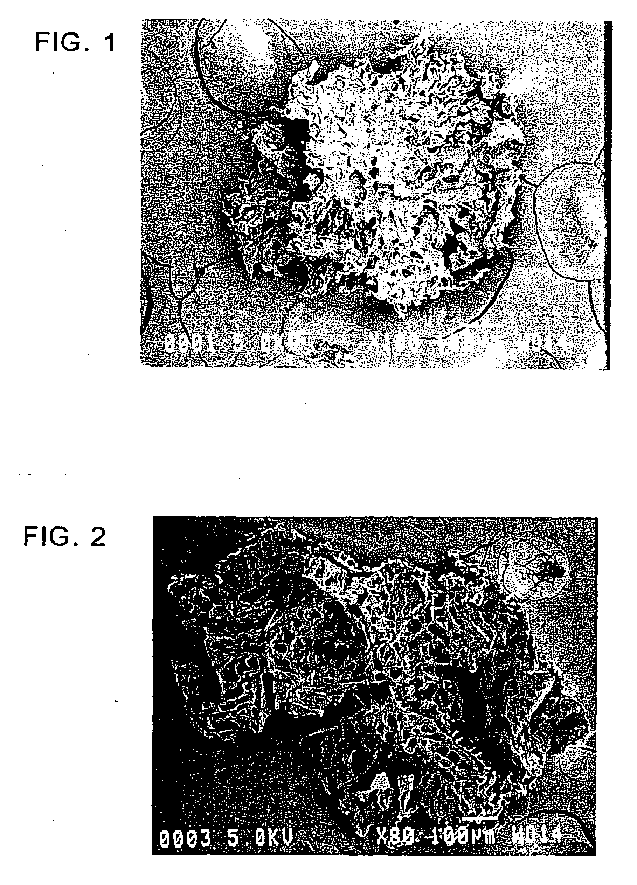

The polymer material containing the sodium chloride approximate-uniformly was obtained, by adding the copolymer of lactic acid and glycolic acid (lactic acid: glycolic acid = 75: 25, the weight average molecular weight was about 250000) into dioxane to have the concentration of 12 wt.%, stirring it by a stirrer to dissolve it, approximate-uniformly mixing a sodium chloride powder (a particle diameter was 300 to 700 µm) with the dioxane solution of the copolymer of lactic acid and glycolic acid being dissolved to have the sodium chloride concentration of about 1.18 g/cm³, pouring it into a mold, freezing it under the condition of -30°C by a freezer (MDf-0281AT made by Sanyo Electric Corporation), and drying it under a reduced pressure for 48 hours by a vacuum dryer (DP43 made by Yamato Scientific Corporation) to remove the dioxane. Then, the bioabsorbable granular porous bone filling material was obtained at the yield of about 86%, by cutting the polymer material to become small pieces, pulverizing the small pieces by a planetary ball mill for 50 minutes, taking the pulverized polymer material into a flask, adding a distilled water to the flask, stirring it for 1 hour to remove sodium chloride, moving it to a petri dish, drying it by the vacuum dryer for 48 hours, and passing it through a sieve. This bioabsorbable granular porous bone filling material had the particle diameter of 300 to 700 µm, and had the average hole diameter of about 5 µm . The scanning electron microscope photograph of this bioabsorbable granular porous bone filling material is shown in Figure 1.

### <Example 2>

The polymer material containing the sodium chloride approximate-uniformly was obtained, by adding the polyglycolic acid (the weight average molecular weight was about 200000) into dichloromethane to have the concentration of 9 wt.%, stirring it by the stirrer to dissolve it, pouring the dichloromethane solution of polyglycolic acid being dissolved into a mold where the sodium chloride powder (the particle diameter was 300 to 700 µm) was already put in to have sodium chloride concentration of about 1.18 g/cm³, freezing it under the condition of -30°C by the freezer (MDf-0281AT made by Sanyo Electric Corporation), and drying it under a reduced pressure for 48 hours by the vacuum dryer (DP43 made by Yamato Scientific Corporation) to remove dichloromethane. Then, the bioabsorbable granular porous bone filling material was obtained at the yield of about 80%, by cutting the polymer material to become small pieces, pulverizing the small pieces by the planetary ball mill for 20 minutes, taking the pulverized polymer material into a flask, adding the distilled water to the flask, stirring it to remove sodium chloride, moving it to the petri dish, drying it by the vacuum dryer for 48 hours, and passing it through the sieve. This bioabsorbable granular porous bone filling material had the particle diameter of 700 to 1400 µm, and the average hole diameter of about 20 µm.

### <Example 3>

The polymer material containing the potassium chloride uniformly was obtained, by adding the copolymer of lactic acid and glycolic acid (lactic acid: glycolic acid = 75: 25, the weight average molecular weight was about 250000) into dioxane to have the concentration of 12 wt.%, stirring it by the stirrer to dissolve it, pouring the dioxane solution of the copolymer of lactic acid and glycolic acid being dissolved into the mold where a potassium chloride powder (the particle diameter was about 400 µm) is already put in to have the potassium chloride concentration of about 1.08 g/cm³, freezing it under the condition of -30°C by the freezer (MDf-0281AT made by Sanyo Electric Corporation), and drying it under a reduced pressure for 48 hours by the vacuum dryer (DP43 made by Yamato Scientific Corporation) to remove dioxane. Then, the bioabsorbable granular porous bone filling material was obtained at the yield of about 86%, by cutting the polymer material to become small pieces, pulverizing the small pieces by the planetary ball mill for 50 minutes, taking the pulverized polymer material into the flask, adding the distilled water to the flask, stirring it to remove potassium chloride, moving it to the petri dish, drying it by the vacuum dryer for 48 hours, and passing it through the sieve. This bioabsorbable granular porous bone filling material had the particle diameter of 300 to 700 µm, and the average hole diameter of about 20 µm.

### <Example 4>

The polymer material containing the citric acid trisodium was obtained, by adding the poly- (L)- lactic acid (the weight average molecular weight was about 250000) into dichloromethane to have the concentration of 6 wt.%, stirring it by the stirrer to dissolve it, approximate-uniformly mixing a citric acid trisodium powder (the particle diameter was about 200 to 500 µm) with the dichloromethane solution of the poly- (L)- lactic acid being dissolved to have the concentration of about 1.02 g/cm³, pouring it into the mold, freezing it under the condition of -30 °C by the freezer (MDf-0281AT made by Sanyo Electric Corporation), and drying it under a reduced pressure for 48 hours by the vacuum dryer (DP43 made by Yamato Scientific Corporation) to remove dichloromethane. Then, the bioabsorbable granular porous bone filling material was obtained at the yield of about 84%, by cutting the polymer material to become small pieces, pulverizing the small pieces by the planetary ball mill for 20 minutes, taking the pulverized polymer material into the flask, adding the distilled water to the flask, stirring it to remove citric acid trisodium, moving it to the petri dish, drying it by the vacuum dryer for 48 hours, and passing it through the sieve. This bioabsorbable granular porous bone filling material had the particle diameter of 700 to 1400 µm, and the average hole diameter of about 25 µm. The scanning electron microscope photograph of this bioabsorbable granular porous bone filling material is shown in Figure 2.

## Claims

1. A bioabsorbable granular porous bone filling material comprising,
a bioabsorbable polymer material having a small hole structure where a hole diameter is 5 to 50 µm, and
having a particle diameter of 100 to 3000 µm.

2. The bioabsorbable granular porous bone filling material as claimed in claim 1,
wherein a bioabsorbable polymer for the polymer material is at least one kind selected from polyglycolic acid, polylactic acid, a copolymer of lactic acid and glycolic acid, poly- ε- caprolactone, a copolymer of lactic acid and e- caprolactone, polyamino acid, polyortho ester, and a copolymer of those.

3. The bioabsorbable granular porous bone filling material as claimed in claim 1 or 2,
wherein a weight average molecular weight of the bioabsorbable polymer is 5000 to 2000000.

4. A production method of the bioabsorbable granular porous bone filling material, the method comprising,
approximate-uniformly mixing a particle-shaped material having a particle diameter of 100 to 2000 µm with a solution, where the bioabsorbable polymer is dissolved with an organic solvent, the particle-shaped material being not dissolved with said organic solvent but dissolvable with a liquid which does not dissolve the bioabsorbable polymer, freezing it,
drying it to remove said organic solvent to produce the polymer material containing the particle-shaped material and having the small hole structure where the hole diameter is 5 to 50 µm, pulverizing said produced polymer material,
dissolving said particle-shaped material with the liquid to be removed, where the liquid does not dissolve the bioabsorbable polymer, and
passing it through a sieve to produce the bioabsorbable granular porous bone filling material having the particle diameter of 100 to 3000 µm.

5. The production method of the bioabsorbable granular porous bone filling material as claimed in claim 4,
wherein at least one kind selected from polyglycolic acid, polylactic acid, a copolymer of lactic acid and glycolic acid, poly-ε- caprolactone, a copolymer of lactic acid and ε- caprolactone, polyamino acid, polyortho ester, and a copolymer of those, is used as the bioabsorbable polymer.

6. The production method of the bioabsorbable granular porous bone filling material as claimed in claim 4 or 5,
wherein a polymer having the weight average molecular weight of 5000 to 2000000 is used as the bioabsorbable polymer.

7. The production method of the bioabsorbable granular porous bone filling material as claimed in claim 4, 5 or 6
wherein at least one kind selected from chloroform, dichloromethane, carbon tetrachloride, acetone, dioxane, and tetrahydrofuran is used as the organic solvent.

8. The production method of the bioabsorbable granular porous bone filling material as claimed in anyone of claims 4 to 7,
wherein a water soluble organic and/or inorganic salt is used as the particle-shaped material, and water is used as the liquid which dissolves the particle-shaped material and does not dissolve the bioabsorbable polymer.

9. The production method of the bioabsorbable granular porous bone filling material as claimed in anyone of claims 4 to 8,
wherein the polymer material is produced by making the concentration of the bioabsorbable polymer of 1 to 20 wt.% and the concentration of the particle-shaped material of 1.0 to 1.5 g/cm³, with respect to the organic solvent.

## Patentansprüche

1. Bioabsorbierbares granuläres poröses Knochenfüllmaterial, umfassend ein bioabsorbierbares Polymermaterial, das eine Struktur kleiner Löcher aufweist, wobei ein Lochdurchmesser 5 bis 50 µm beträgt, und das einen Teilchendurchmesser von 100 bis 3.000 µm aufweist.

2. Bioabsorbierbares granuläres poröses Knochenfüllmaterial, wie in Anspruch 1 beansprucht, wobei ein bioabsorbierbares Polymer für das Polymermaterial mindestens eines ist, ausgewählt aus Polyglykolsäure, Polymilchsäure, einem Copolymer von Milchsäure und Glykolsäure, Poly-ε-caprolacton, einem Copolymer von Milchsäure und ε-Caprolacton, Polyaminosäure, Polyorthoester und einem Copolymer von diesen.

3. Bioabsorbierbares granuläres poröses Knochenfüllmaterial, wie in Anspruch 1 oder 2 beansprucht, wobei ein gewichtsmittleres Molekulargewicht des bioabsorbierbaren Polymers 5.000 bis 2.000.000 beträgt.

4. Herstellungsverfahren für das bioabsorbierbare granuläre poröse Knochenfüllmaterial, wobei das Verfahren umfaßt:
das annähernd einheitliche Mischen eines teilchenförmigen Materials mit einem Teilchendurchmesser von 100 bis 2.000 µm mit einer Lösung, wobei das bioabsorbierbare Polymer durch ein organisches Lösungsmittel gelöst wird, wobei das teilchenförmige Material nicht durch das organische Lösungsmittel gelöst wird, aber durch eine Flüssigkeit lösbar ist, welche nicht das bioabsorbierbare Polymer löst,
das Einfrieren dieses,
das Trocknen dieses, um das organische Lösungsmittel zu entfernen, unter Herstellung des Polymermaterials, welches das teilchenförmige Material enthält und die Struktur kleiner Löcher aufweist, wobei der Lochdurchmesser 5 bis 50 µm beträgt,
das Pulverisieren des hergestellten Polymermaterials,
das Lösen des teilchenförmigen Materials durch die Flüssigkeit, die entfernt wird, wobei die Flüssigkeit nicht das bioabsorbierbare Polymer löst, und
das Leiten dieses durch einen Sieb unter Herstellung des bioabsorbierbaren granulären porösen Knochenfüllmaterials mit einem Teilchendurchmesser von 100 bis 3.000 µm.

5. Herstellungsverfahren für das bioabsorbierbare granuläre poröse Knochenfüllmaterials, wie in Anspruch 4 beansprucht, wobei mindestens eine, ausgewählt aus Polyglykolsäure, Polymilchsäure, einem Copolymer von Milchsäure und Glykolsäure, Poly-ε-caprolacton, einem Copolymer von Milchsäure und ε-Caprolacton, Polyaminosäure, Polyorthoester und einem Copolymer von diesen, als das bioabsorbierbare Polymer verwendet wird.

6. Herstellungsverfahren für das bioabsorbierbare granuläre poröse Knochenfüllmaterial, wie in Anspruch 4 oder 5 beansprucht, worin ein Polymer mit dem gewichtsmittleren Molekulargewicht von 5.000 bis 2.000.000 als das bioabsorbierbare Polymer verwendet wird.

7. Herstellungsverfahren für das bioabsorbierbare granuläre poröse Knochenfüllmaterial, wie in Anspruch 4, 5 oder 6 beansprucht, wobei mindestens eines, ausgewählt aus Chloroform, Dichlormethan, Tetrachlorkohlenstoff, Aceton, Dioxan und Tetrahydrofuran, als das organische Lösungsmittel verwendet wird.

8. Herstellungsverfahren für das bioabsorbierbare granuläre poröse Knochenfüllmaterial, wie in einem der Ansprüche 4 bis 7 beansprucht, wobei ein wasserlösliches organisches und/oder anorganisches Salz als das teilchenförmige Material verwendet wird, und Wasser als die Flüssigkeit verwendet wird, welche das teilchenförmige Material löst und das bioabsorbierbare Polymer nicht löst.

9. Herstellungsverfahren für das bioabsorbierbare granuläre poröse Knochenfüllmaterial, wie in einem der Ansprüche 4 bis 8 beansprucht, wobei das Polymermaterial durch Einstellen der Konzentration des bioabsorbierbaren Polymers auf 1 bis 20 Gew.-% und der Konzentration des teilchenförmigen Materials auf 1,0 bis 1,5 g/cm³, bezogen auf das organische Lösungsmittel, hergestellt wird.

## Revendications

1. Matière d'obturation osseuse poreuse granulaire bioabsorbable comprenant une matière polymère bioabsorbable ayant une structure à petits trous dans laquelle le diamètre des trous est de 5 à 50 µm et ayant un diamètre des particules de 100 à 3000 µm.

2. Matière d'obturation osseuse poreuse granulaire bioabsorbable selon la revendication 1, dans laquelle un polymère bioabsorbable pour la matière polymère est au moins un type choisi parmi l'acide polyglycolique, l'acide polylactique, un copolymère d'acide lactique et d'acide glycolique, la poly-ε-caprolactone, un copolymère d'acide lactique et d'ε-caprolactone, un polyaminoacide, un polyortho-ester et un copolymère de ceux-ci.

3. Matière d'obturation osseuse poreuse granulaire bioabsorbable selon la revendication 1 ou 2, dans laquelle la masse moléculaire moyenne en poids du polymère bioabsorbable est de 5000 à 2 000 000.

4. Procédé de production de la matière d'obturation osseuse poreuse granulaire bioabsorbable, ledit procédé comprenant les étapes consistant à
mélanger de façon approximativement uniforme une matière sous forme de particules, ayant un diamètre des particules de 100 à 2000 µm, avec une solution, dans lequel le polymère bioabsorbable est dissous avec un solvant organique, la matière sous forme de particules n'étant pas dissoute avec ledit solvant organique mais pouvant être dissoute avec un liquide qui ne dissout pas le polymère bioabsorbable, la congeler, la sécher pour éliminer ledit solvant organique pour produire la matière polymère contenant la matière sous forme de particules et ayant la structure à petits trous dans laquelle le diamètre des trous est de 5 à 50 µm,
pulvériser ladite matière polymère produite,
dissoudre ladite matière sous forme de particules avec le liquide à éliminer, le liquide ne dissolvant le polymère bioabsorbable, et
la faire passer à travers un tamis pour produire la matière d'obturation osseuse poreuse granulaire bioabsorbable ayant le diamètre de particules de 100 à 3000 µm.

5. Procédé de production de la matière d'obturation osseuse poreuse granulaire bioabsorbable selon la revendication 4, dans lequel au moins un type choisi parmi l'acide polyglycolique, l'acide polylactique, un copolymère d'acide lactique et d'acide glycolique, la poly-ε-caprolactone, un copolymère d'acide lactique et d'ε-caprolactone, un polyaminoacide, un polyorthoester et un copolymère de ceux-ci est utilisé comme polymère bioabsorbable.

6. Procédé de production de la matière d'obturation osseuse poreuse granulaire bioabsorbable selon la revendication 4 ou 5, dans lequel on utilise un polymère ayant la masse moléculaire moyenne en poids de 5000 à 2 000 000 comme polymère bioabsorbable.

7. Procédé de production de la matière d'obturation osseuse poreuse granulaire bioabsorbable selon la revendication 4, 5 ou 6, dans lequel on utilise au moins un type choisi parmi le chloroforme, le dichlorométhane, le tétrachlorure de carbone, l'acétone, le dioxanne et le tétrahydrofuranne comme solvant organique.

8. Procédé de production de la matière d'obturation osseuse poreuse granulaire bioabsorbable selon l'une quelconque des revendications 4 à 7, dans lequel on utilise un sel organique et/ou inorganique soluble dans l'eau comme matière sous forme de particules et on utilise de l'eau comme liquide qui dissout la matière sous forme de particules et qui ne dissout pas le polymère bioabsorbable.

9. Procédé de production de la matière d'obturation osseuse poreuse granulaire bioabsorbable selon l'une quelconque des revendications 4 à 8, dans lequel on prépare la matière polymère en amenant la concentration du polymère bioabsorbable de 1 à 20 % en poids et la concentration de la matière sous forme de particules de 1,0 à 1,5 g/cm³ par rapport au solvant organique.
